# EUROPÄISCHE PATENTSCHRIFT

(11) **EP 1 250 132 B1**
(45) Veröffentlichungstag und Bekanntmachung des Hinweises auf die Patenterteilung: **30.03.2005**
(21) Anmeldenummer: 01902340.7
(22) Anmeldetag: 19.01.2001
(51) Int. Cl.: A61K 31/19, A61K 9/28

(54) **IBUPROFEN-WIRKSTOFFZUBEREITUNG**
IBUPROFEN CONTAINING ACTIVE AGENT PREPARATION
PREPARATION A PRINCIPE ACTIF IBUPROFENE

(30) Priorität: 28.01.2000 DE 10003757
(43) Veröffentlichungstag der Anmeldung: 23.10.2002
(73) Patentinhaber: BASF AKTIENGESELLSCHAFT, 67056 Ludwigshafen (DE)
(72) Erfinder: EINIG, Heinz, 67433 Neustadt (DE)
(86) Internationale Anmeldenummer: PCT/EP2001/000602
(87) Internationale Veröffentlichungsnummer: WO 2001/054683

(56) Entgegenhaltungen:
- EP-A- 0 396 404
- EP-A- 0 456 720
- WO-A-93/04676
- WO-A-95/35104
- WO-A-99/17748
- US-A- 5 254 728

## Beschreibung

Die vorliegende Erfindung betrifft eine Wirkstoffzubereitung, enthaltend Ibuprofen, ein Verfahren zu deren Herstellung, deren Verwendung zur Herstellung von Arzneimittelzubereitungen und festen Darreichungsformen, sowie Arzneimittelzubereitungen und feste Darreichungsformen, enthaltend die Ibuprofen-Wirkstoffzubereitung.

Ibuprofen, 2-(4-isobutylphenyl)-propionsäure ist ein bekannter Wirkstoff mit anti-entzündlicher, analgetischer und antipyretischer Wirkung, der hauptsächlich zur Behandlung von Schmerzen und zur Entzündungshemmung eingesetzt wird.

Aufgrund der schlechten Komprimierbarkeit und des niedrigen Schmelzpunktes der bekannten Ibuprofen-Kristallmodifikationen weisen racemisches Ibuprofen und insbesondere das besser und schneller wirksame S-Enantiomer schlechte Tablettierungseigenschaften auf und sind nur unter Zuhilfenahme großer Mengen pharmazeutischer Hilfsstoffe in ausreichend stabile, feste Darreichungsformen, wie beispielsweise Tabletten überführbar.

Ibuprofen wird in der Regel in Tabletten mit 200 oder 400 mg Wirkstoff/Tablette, bei rheumatischen Beschwerden allerdings auch in Dosen bis 600 und 800 mg Wirkstoff/Tablette verabreicht.

Durch das Zumischen großer Mengen pharmazeutischer Hilfsstoffe ist die Wirkstoffkonzentration in den festen Darreichungsformen auf ca. 30 bis 40 Gew.-% begrenzt. Dies führt einerseits zu höheren Produktionskosten pro Menge Wirkstoff und hat den weiteren Nachteil, daß feste Darreichungsformen mit hohen Wirkstoffdosen aufgrund ihrer Größe kaum mehr schluckbar sind.

Durch den weiteren Verarbeitungsschritt einer Naß- oder Trockengranulierung der Arzneimittelzubereitung vor der Tablettierung kann die Menge der pharmazeutischen Hilfsstoffe reduziert werden, so daß in den festen Darreichungsformen Wirkstoffkonzentrationen bis zu 66% erreicht werden.

Dieser Schritt ist allerdings sehr zeit- und kostenaufwendig. Ferner hat das Verfahren den Nachteil, daß eine Rezeptur für feste Darreichungsformen mit hohen Wirkstoffdosen nicht flexibel auf kleinere feste Darreichungsformen mit geringeren Wirkstoffdosen übertragbar ist. Die kleineren festen Darreichungsformen weisen in der Regel nicht mehr genügend Härte auf.

Zur Reduzierung der Kosten ist heutzutage ein superschnelles und flexibles Tablettieren Stand der Technik. Dazu ist es notwendig, daß Arzneimittelzubereitungen flexibel für feste Darreichungsformen unterschiedlicher Größe und Wirkstoffdosis einsetzbar sind.

Ein kostensparendes Verfahren zur Herstellung von festen Darreichungsformen ist das sogenannte Direkttablettieren. Dabei werden alle Bestandteile einer Tablette in einem einzigen Schritt zur tablettierfertigen Masse gemischt und anschließend zur fertigen Tablette gepreßt. Dadurch kann der Schritt einer Vorgranulierung vermieden werden.

Dazu muß Ibuprofen in eine Form überführt werden, die direkt tablettierbar ist.

Es ist bekannt, Ibuprofen in eine Ibuprofenhaltige Wirkstoffzubereitung zu überführen, die direkt mit den restlichen Bestandteilen der Tablette wie beispielsweise pharmazeutischen Hilfsmitteln tablettiert werden kann.

Vorteilhafte Ibuprofenhaltige Wirkstoffzubereitungen zur Direkttablettierung weisen möglichst viele der folgenden Eigenschaften auf:
- Die Wirkstoffzubereitung läßt sich direkt zu festen Darreichungsformen mit ausreichender Härte pressen.
- Die Wirkstoffzubereitung weist eine hohen Ibuprofenkonzentration auf.
- Die aus der Wirkstoffzuberereitung hergestellten festen Darreichungsformen weisen eine hohe Ibuprofenkonzentration auf, d.h. zur Herstellung von vorteilhaften festen Darreichungsformen aus der Wirkstoffzuberereitung sind möglichst wenige weitere pharmazeutische Hilfsmittel nötig.
- Die aus der Wirkstoffzuberereitung hergestellten festen Darreichungsformen weisen eine ausreichende Härte und Abriebfestigkeit auf.
- Die aus der Wirkstoffzuberereitung hergestellten festen Darreichungsformen zerfallen und setzen den Wirkstoff Ibuprofen in Lösung in der gewünschten Zeit frei (Instant-Release/Sustained-Release)
- Die aus der Wirkstoffzuberereitung hergestellten festen Darreichungsformen sind lagerstabil.
- Die aus der Wirkstoffzuberereitung gemischten Arzneimittelzubereitungen sind flexibel für unterschiedliche Größen der Darreichungsformen verwendbar und erlauben ein superschnelles Tablettieren.

WO 89/02266 beschreibt ein Verfahren zur Herstellung einer tablettierfähigen Arzneimittelformulierung enthaltend Ibuprofen durch Übersprühen einer wäßrigen Mischung aus Ibuprofen und Natriumcarboxymethylcellulose mit einer Dispersion aus pregelatinierter Stärke in einem Fließbett-Granulationstrockenverfahren, anschließender Trocknung und weiterem Zumischen eines Gleitmittels, wobei weitere pharmazeutische Hilfsstoffe vor und nach dem Übersprühen zugemischt werden können. Die im Verfahren hergestellte Wirkstoffzubereitung erreicht eine maximale Ibuprofenkonzentration von 88,5 Gew.-%. Die daraus resultierenden Arzneimittelzubereitungen und Tabletten weisen dementsprechend eine maximale Ibuprofenkonzentration von nur 85 Gew.-% auf. Tabletten mit optimaler Härte und Wirkstofffreisetzung die nach diesem Verfahren hergestellt werden weisen sogar nur eine Ibuprofenkonzentration von maximal 63 Gew.-% auf. Das Verfahren und die damit hergestellte Arzneimittelformulierung hat somit den Nachteil, daß keine Tabletten mit höheren Ibuprofenkonzentrationen als 85 Gew.-% hergestellt werden können.

EP 456 720 beschreibt eine 85 bis 99 Gew.-%ige Ibuprofenhaltige Wirkstoffzubereitung die nach Zugabe weiterer pharmazeutischer Hilfsstoffe direkt tablettiert werden kann. Dazu wird Ibuprofen mit einem Bindemittel (jedoch kein Polyvinylpyrrolidon) vermischt und fluidisiert und mit einer Dispersion aus Polyvinylpyrrolidon (PVP) und weiteren Bindemitteln in einem Sprühtrocknungsverfahren granuliert. Dieses Verfahren und die damit hergestellte Wirkstoffzubereitung weist den Nachteil auf, daß unvernetztes Polyvinylpyrrolidon als Granuliermittel bzw. als filmbildendes Bindemittel im Granulierverfahren verwendet wird. Unvernetztes Polyvinylpyrrolidon bildet mit Ibuprofen bei Temperaturen von 30 bis 40°C eine eutektische Phase, die bei Raumtemperatur flüssig wird. Diese führt zu hohen Belägen an den Tablettenstempeln, so daß ein superschnelles Tablettieren nicht mehr möglich ist. Ferner weisen Tabletten die aus dieser mit unvernetztem PVP granulierten Wirkstoffzubereitung hergestellt werden den Nachteil einer geringeren Lagerfähigkeit auf. Bei 30 bis 40°C härten die Tabletten nach, so daß die Freisetzungsrate des Wirkstoffs reduziert wird.

Es war daher die Aufgabe der vorliegenden Erfindung eine weitere Ibuprofenhaltige Wirkstoffzubereitung zur Verfügung zu stellen, die die vorstehend erwähnten Nachteile nicht mehr aufweist und möglichst viele der vorstehend erwähnten vorteilhaften Eigenschaften erfüllt.

Dementsprechend wurde ein Verfahren zur Herstellung einer Wirkstoffzubereitung enthaltend Ibuprofen gefunden, indem man Ibuprofen oder dessen Salze mit einem Bindemittel außer Polyvinylpyrollidon überzieht, wobei bezogen auf das Trockengewicht der Wirkstoffzubereitung der Anteil des Bindemittels 0,1 bis 10 Gew.-% und der Anteil an Ibuprofen oder dessen Salze 90 bis 99,9 Gew.-% beträgt.

Unter Ibuprofen werden sowohl racemisches Ibuprofen als auch die isolierten Enantiomere R-(-)-Ibuprofen und insbesondere das schneller und besser wirksame S-(+)-Ibuprofen verstanden.

Als Salze des Ibuprofens kommen insbesondere die Alkali- und Erdalkalimetallsalze sowie die Salze von Ibuprofen mit basischen Aminosäuren wie beispielsweise Lysin in Frage. Besonders bevorzugte Salze sind Ibuprofen-Natrium, Ibuprofen-Calcium und Ibuprofen-Lysin.

Die Partikelgröße des eingesetzten Ibuprofens ist nicht kritisch. Typischerweise beträgt sie 10 bis 100 µm, insbesondere 20 bis 80 µm. Die Reinheit des eingesetzten Ibuprofens ist für die Erfindung ebenfalls nicht kritisch, sollte aber im Hinblick auf eine hohe Wirkstoffkonzentration in der festen Darreichungsform möglichst hoch sein, insbesondere höher als 96 % oder 99 %.

Unter Bindemittel werden auch Mischungen von Bindemitteln verstanden. Bei der Herstellung der Wirkstoffzubereitung können alle bekannten Bindemittel, jedoch kein Polyvinylpyrrolidon (PVP), insbesondere kein unvernetztes PVP verwendet werden.

Als Bindemittel kommen beispielsweise Cellulosen oder Cellulosederivate, Stärke oder Stärkederivate, Gelatine, Alginate-Suspensionen oder Zuckerlösungen in Frage.

Bevorzugte Bindemittel sind in Wasser lösliche, suspendierbare oder dispergierbare Bindemittel. Besonders bevorzugte Bindungsmittel sind Hydroxymethylpropylcellulosen, insbesondere mit einem mittleren Molekulargewicht, das etwa dem Handelstyp Pharmacoat® 603 oder 606, der Firma Shinetsu Chemical Comp., Tokyo, Japan oder höherviskosen Graden entspricht, Methyl-Cellulose, Natriumcarboxymethyl-Cellulose, Hydroxypropylcellulose oder Gelatine.

Das Überziehen des Wirkstoffs Ibuprofen mit dem Bindemittel kann prinzipiell nach allen bekannten Arten durchgeführt werden. Dazu wird in der Regel das Bindemittel in flüssiger oder geschmolzener Form oder in einer Lösung, Suspension oder Dispersion intensiv mit dem zu überziehenden Wirkstoff in Kontakt gebracht und die entstehenden Granulate anschließend, gegebenenfalls nach einer Klassifizierung und Homogenisierung, getrocknet. Anschließend können die getrockneten Granulate, beispielsweise durch entsprechende Siebe, weiter klassifiziert oder homogenisiert werden.

Das Aufbringen der Lösung, Suspension oder Dispersion des Bindemittels kann aus organischer oder wäßriger Lösung erfolgen. In einer bevorzugten Ausführungsform werden wässerige Lösungen, Suspensionen oder Dispersionen des Bindemittels verwendet.

Das Inkontaktbringen kann durch Mischen des Wirkstoffs mit der Schmelze oder Lösung, Suspension oder Dispersion des Bindemittels, beispielsweise in einem üblichen Feuchtmischer, erfolgen.

Das Feuchtmischen kann auch beispielsweise so erfolgen, daß der wirkstoff und das Bindemittel trocken gemischt werden und anschließend mit einem organischem Lösungsmittel oder Wasser fluidisiert und verknetet werden.

Bei der Feuchtmischung erfolgt die Trocknung der erhaltenen Granulate separat, beispielsweise nach einer Klassifizierung oder Homogenisierung des feuchten Granulats in einem Trockner, wie beispielsweise einem IR-, Umluft- oder Wirbelschichttrockner.

Das Inkontaktbringen kann aber auch durch Versprühen der Schmelze oder Lösung, Suspension oder Dispersion des Bindemittels auf oder mit dem Wirkstoff in einem Fließ- oder Wirbelbett erfolgen.

Dabei kann das Inkontaktbringen und Trocknen in einem Schritt erfolgen, beispielsweise in Wirbelschichtgranulatoren.

In einer bevorzugten Ausführungsform des Verfahrens erfolgt das Überziehen von Ibuprofen oder dessen Salze mit dem Bindemittel in einem Feuchtmischer, beispielsweise mit Flugscharmischer und Zerhacker und anschließender Trocknung oder in Wirbelschichtgranulatoren mit Bottom- oder Top-Spraying, insbesondere in Granulatoren vom Typ Wurster oder Glatt-Zeller.

Wirtschaftlich am ökonomischten sind Feuchtmischer, da der Wirkstoff und das Bindemittel zunächst nur gemischt werden, dann mit Wasser oder einem organischen Lösungsmittel befeuchtet und geknetet werden. Nach Herstellung eines homogenen feuchten Granulates kann in einen herkömmlichen Trockner (IR-, Umluftoder Wirbelschichttrockner) getrocknet werden. Nach der Homogenisierung erfolgt eine Klassifizierung über Siebe. Bei dieser Methode erreicht man einen großen Durchsatz.

Bei der Herstellung der Ibuprofenhaltigen Wirkstoffzubereitung beträgt der Anteil des Bindemittels 0,1 bis 10 Gew.-% und der Anteil an Ibuprofen oder dessen Salze 90 bis 99,9 Gew.-%, jeweils bezogen auf das Trockengewicht der Wirkstoffzubereitung.

In einer bevorzugten Ausführungsform beträgt der Anteil des Bindemittels 0,2 bis 6 Gew.-% und der Anteil an Ibuprofen oder dessen Salze 94 bis 99,8 Gew.-%, jeweils bezogen auf das Trockengewicht der Wirkstoffzubereitung.

In einer besonders bevorzugten Ausführungsform beträgt der Anteil des Bindemittels 0,3 bis 3,5 Gew.-% und der Anteil an Ibuprofen oder dessen Salze 96,5 bis 99,7 Gew.-%, jeweils bezogen auf das Trockengewicht der Wirkstoffzubereitung.

Dem zu überziehenden Ibuprofen oder dessen Salze können nach oder vorzugsweise vor dem Überziehen mit Bindemittel bis zu 1,5 Gew.-% Adsorptionsmittel, wie beispielsweise Pharmasorb^{(R)} der Firma Minerals & Chemicals Div., Menlo PArk, Edison, N.J. 08817, USA, oder hochdisperses Siliciumdioxid, wie beispielsweise Aerosil® 200 zugesetzt werden.

Im Falle einer Zusetzung von Adsorptionsmittel ist hochdisperses Siliciumdioxid, wie beispielsweise Aerosil® 200, Firma Degussa, Frankfurt/Main, Deutschland bevorzugt.

Die vorliegende Erfindung betrifft dementsprechend eine ibuprofenhaltige Wirkstoffzubereitung, erhältlich nach dem vorstehend beschriebenen Verfahren.

Im Gegensatz zu einer rein physikalischen Mischung, die 90 bis 99,9 Gew.-% Ibuprofen und 0,1 bis 10 Gew.-% Bindemittel jeweils bezogen auf die Trockenmasse der Mischung enthält, läßt sich die erfindungsgemäße wirkstoffzubereitung in Preßlinge überführen, die eine Härte von größer 25 N aufweisen.

Die Erfindung betrifft deshalb ferner eine Polyvinylpyrrolidonfreie Wirkstoffzuberereitung, enthaltend

| | |
|---|---|
| 90,0 bis 99,9 Gew.-% | Ibuprofen oder dessen Salze, |
| 0,1 bis 10,0 Gew.-% | Bindemittel außer Polyvinylpyrrolidon und |
| 0,1 bis 1,5 Gew.-% | hochdispersem Siliciumdioxid, |

jeweils bezogen auf das Trockengewicht der Wirkstoffzubereitung,
wobei die aus dieser Wirkstoffzubereitung mit einer Preßkraft von 4,7 bis 5,3 kN hergestellten Preßlinge mit einem Ibuprofenwirkstoffgehalt von 200 mg und einem Durchmesser von 9 mm eine Härte von mindestens 25 N aufweisen.

Unter der Härte der runden Preßlinge wird die radiale Bruchkraft verstanden.

Insbesondere betrifft die Erfindung eine Wirkstoffzuberereitung, bestehend aus

| | |
|---|---|
| 90,0 bis 99,9 Gew.-% | Ibuprofen oder dessen Salze, |
| 0,1 bis 10,0 Gew.-% | Bindemittel außer Polyvinylpyrrolidon und |
| 0 bis 1,5 Gew.-% | hochdispersem Siliciumdioxid, |

jeweils bezogen auf das Trockengewicht der Wirkstoffzubereitung,
wobei die aus dieser Wirkstoffzubereitung mit einer Preßkraft von 4,7 bis 5,3 kN hergestellten Preßlinge mit einem Ibuprofenwirkstoffgehalt von 200 mg und einem Durchmesser von 9 mm eine Härte von mindestens 25 N aufweisen.

In diesem Fall addieren sich die Gewichtsprozentwerte der Bestandteile der Wirkstoffzubereitung zu 100 Gew.-%.

Die erfindungsgemäße Wirkstoffzubereitung kann durch Zugabe geringer Mengen pharmazeutischer Hilfsstoffe in eine Arzneimittelzubereitung überführt werden, die direkt, beispielsweise durch Verpressen in eine feste Darreichungsform mit hoher Ibuprofenkonzentration, optimaler Härte und optimaler Wirkstoff-Freisetzung überführt werden kann.

Dementsprechend betrifft die vorliegende Erfindung ferner die Verwendung der erfindungsgemäßen Wirkstoffzubereitung Herstellung von Arzneimittelzubereitungen oder festen Darreichungsformen.

Die erfindungsgemäße Arzneimittelzubereitung, enthält die erfindungsgemäße ibuprofenhaltige Wirkstoffzubereitung und vorzugsweise zusätzlich Tabletten-Hilfsstoffe, die den jeweiligen Bedürfnissen der schnellen oder langsamen Wirkstoff-Freisetzung oder weiteren galenischen Bedürfnissen angepaßt sind.

### Die Arzneimittelzubereitung kann zusätzlich zur Wirkstoffzubereitung weitere

Bindemittel außer unvernetztes PVP, wie beispielsweise Hydroxymethylpropylcellulosen oder Hydroxypropylcellulosen,

Sprengmittel, wie beispielsweise AcDiSOl®, der Firma FMC Corp. Philadelphia, PA 19103 oder Primojel® der Firma VZB, Korg van de Zaan, Foxkol, Niederlande,

Trockenbindemittel, wie beispielsweise mikrokristalline Cellulose,

Hydrophilisierungsmittel, wie beispielsweise Cremophor® RH 40, Firma BASF Aktiengesellschaft, Ludwigshafen, Deutschland,

Adsorptionsmittel, wie beispielsweise Verosil® der Firma Degussa, Frankfurt, Deutschland,

### Gleitmittel, wie beispielsweise Magnesiumstearat

oder Hilfsmittel die eine verzögerte Freisetzung des Wirkstoffs bewirken, wie beispielsweise Kollidon^{(R)} SR der Firma BASF Aktiengesellschaft, Ludwigshafen, Deutschland oder hochpolymere Hydroxypropylmethylcellulose vom Typ k4M oder E4M der Firma Colorcan, 65510 Idstein, Deutschland, enthalten.

In einer bevorzugten Ausführungsform enthalten die Arzneimittelzubereitungen Hilfsstoffe, die eine verzögerte Wirkstoffabgabe aus festen Darreichungsformen bewirken.

Die Menge der in den Arzneimittelzubereitungen zusätzlich zur Wirkstoffzubereitung enthaltenen Hilfsstoffe ist nicht kritisch und beträgt je nach Anforderung typischerweise für

| | |
|---|---|
| Sprengmittel | 0 bis 5 Gew.-%, |
| Trockenbindemittel | 0 bis 10 oder 15 Gew.-%, |
| Hydrophilisierungsmitte10 | bis 1 Gew.-%, |
| Adsorptionsmittel | 0 bis 1 Gew.-%, |
| Gleitmittel | 0 bis 1 Gew.-%, |

Hilfsmittel die eine verzögerte Freisetzung des Wirkstoffs bewirken, 0 bis 40 Gew.-%,
während die Menge der erfindungsgemäßen Wirkstoffzubereitung in der Arzneimittelzubereitung typischerweise 30 bis 96 Gew.-% beträgt, jeweils bezogen auf das Trockengewicht der Arzneimittelformulierung und damit auch bezogen auf das Trockengewicht der daraus hergestellten festen Darreichungsform.

Die Gewichtsprozente der Bestandteile der Arzneimittelzubereitung oder der festen Darreichungsformen addieren sich zu 100 Gew.-%.

Besonders vorteilhaft werden der Wirkstoffzubereitung in der Arzneimittelzubereitung allerdings nur geringe Mengen an Hilfsstoffen zugemischt, da dadurch feste Darreichungsformen mit hohen Wirkstoffkonzentrationen hergestellt werden können.

Je nach Ibuprofenkonzentration in der Wirkstoffzubereitung und der Menge der zugesetzten Hilfsstoffe in der Arzneimittelzubereitungen können Arzneimittelzubereitungen und daraus feste Darreichungsformen, vorzugsweise Tabletten, mit optimaler Härte und optimaler Wirkstoff-Freisetzung hergestellt werden, die eine Ibuprofenkonzentration größer 80 Gew-%, insbesondere größer 90 Gew.-% oder sogar größer 94 Gew.-%, jeweils bezogen auf das Trockengewicht der Arzneimittelformulierung bzw. der festen Darreichungsform, aufweisen.

Bei diesen vorteilhaften festen Darreichungsformen beträgt die Summe der Anteile an Bindemittel oder Zusatzstoffen in der Wirkstoffzubereitung und der in der Arzneimittelzubereitung zusätzlich beigemischten Hilfsstoffe dementsprechend maximal 20 Gew.-%, insbesondere weniger als 10 Gew.-% oder sogar weniger als 6 Gew.-%.

Zusätzlich zur ibuprofenhaltigen Wirkstoffzubereitung kann die Arzneimittelzubereitung und die daraus hergestellte feste Darreicungsform neben den gegebenenfalls zugesetzten Hilfsstoffen weitere Wirkstoffe, wie beispielsweise Pseudoephedrinhydrochlorid oder -sulfat, Codeinverbindungen, wie Dihydrocodeinhydrogentatrat enthalten oder andere Wirkstoffe für die eine Kombination mit Ibuprofen pharmakologisch sinnvoll ist, enthalten.

Die Menge des zugesetzten weiteren Wirkstoffs ist nicht kritisch und kann je nach Wirkstoff und Anwendungsbereich typischer Weise 1 bis 80 Gew.-% betragen, bezogen auf die Trockenmasse der Arzneimittelformulierung, bzw. der festen Darreichungsform.

Typischerweise enthält eine feste Darreichungsform, enthaltend eine erfindungsgemäße ibuprofenhaltige Wirkstoffzubereitung und eine Codeinverbindung eine Codeindosis von 10 mg.

Die vorstehend beschriebenen erfindungsgemäßen Arzneimittelzubereitungen, enthaltend die erfindungsgemäße ibuprofenhaltige Wirkstoffzubereitung können direkt zur Herstellung von festen Darreichungsformen verwendet werden.

Die Überführung der Arzneimittelzubereitungen in feste Darreichungsformen kann in an sich bekannter Weise durch alle üblichen Methoden erfolgen.

In einer bevorzugten Ausführungsformen eines Verfahrens zur Herstellung von festen Darreichungsformen wird die erfindungsgemäße Wirkstoffzubereitung oder die erfindungsgemäße Arzneimittelzubereitung, enthaltend die Wirkstoffzubereitung zu einer festen Form komprimiert. Vorzugsweise wird die erfindungsgemäße Arzneimittelzubereitung, enthaltend die erfindungsgemäße Wirkstoffzubereitung in eine Tablettenform gepreßt, also direkt tablettiert.

Bei der Direkttablettierung haben die aus den erfindungsgemäßen Arzneimittelzubereitungen hergestellten festen Darreichungsformen die gleiche Zusammensetzung wie die Arzneimittelzubereitung.

Die erhaltenen festen Darreichungsformen können gegebenenfalls mit Überzugsmitteln, wie beispielsweise Filmen aus Pharmacoat® 603 der Firma Shinetsu mit Polyethylenglykolen als Weichmacher überzogen werden.

Dementsprechend betrifft die vorliegende Erfindung ferner feste Darreichungsformen, vorzugsweise Tabletten, enthaltend die erfindungsgemäße Wirkstoffzubereitung oder die erfindungsgemäße Arzneimittelzubereitung.

Die erfindungsgemäßen festen Darreichungsformen können eine Ibuprofenkonzentration größer 75 Gew.-%, insbesondere größer 90 Gew.-% oder sogar größer 94 Gew.-% aufweisen.

Gegenüber dem Stand der Technik weist die vorliegende Erfindung folgende Vorteile auf:
- Durch die hohe Ibuprofenkonzentration in der direkt tablettierbaren Wirkstoffzubereitungen können hohe Ibuprofenkonzentrationen in den festen Darreichungsformen erreicht werden.
- Die zugrundeliegende Wirkstoffzubereitung ist frei an unvernetztem PVP, so daß eine superschnelles Tablettieren möglich ist und die erhaltenen Tabletten lagerstabil sind.
- Die aus den erfindungsgemäßen Arzneimittelzubereitungen gepreßten Tabletten weisen bei nur geringen Preßkräften eine große und ausreichende Härte mit vernachlässigbarer Friabilität und eine sehr schnelle Wirkstoff-Freisetzung auf, wenn keine Hilfsmittel enthalten sind, die eine Freisetzung verzögern sollen.
- Die erfindungsgemäße Wirkstoffzubereitung kann in Kombination mit anderen Wirkstoffen direkt tablettiert werden.
- Die erfindungsgemäße Arzneimittelzubereitung ist direkt zur Herstellung von Tabletten unterschiedlicher Größe und damit flexibel zur Herstellung von Tabletten mit unterschiedlichen Ibuprofendosen verwendbar.

Die nachstehenden Beispiele erläutern die Erfindung, wobei die Auswahl der Beispiele nicht imitierend ist.

### Beispiel 1

### Ibuprofenhaltigen Wirkstoffzubereitungen

### 1.1 Hydroxypropylmethylcellulose (HPMC) als Bindemittel

### 1.1.1 Überziehen durch Feuchtmischung und Trocknung

9,72 kg Ibuprofen (feines Pulver) wurden mit 240 g Hydroxypropylmethylcellulose und 40 g Aerosil® 200 intensiv in einem Lödige® Mischer (Granulator, Firma Lödige, Paderborn) gemischt und unter leichtem Rühren des Bodenpropellers und des Zerhackers bei Stufe 1 mit 1,50 kg destillierten Wasser befeuchtet und weitere 5 Minuten intensiv gerührt. Danach wurde das feuchte Gut durch ein Sieb der Maschenweite 3 bis 4 mm getrieben und in einem Wirbelschichttrockner (Fa. Glatt) bei einer Zulufttemperatur von 50 bis 60°C in 2 Portionen getrocknet. Es erfolgte eine Siebung mit Sieben kleiner 800 µm. Nach einer Laserbeugungspartikelmessung waren Agglomerate unter 80 bis 90 µm nur in wenigen Prozentanteilen von 2 bis 5 % enthalten.

Zusammensetzung der Wirkstoffzubereitung 1.1.1, bezogen auf das Trockengewicht:

| | |
|---|---|
| Ibuprofen | 97,2 Gew.-% |
| Hydroxypropylmethylcellulose | 2,4 Gew.-% |
| Aerosil 200 | 0,4 Gew.-% |

### 1.1.2 Überziehen durch Wirbelschichtgranulation (Typ Wurster)

2,375 kg Ibuprofen (feines Pulver) wurden mit 15 g Aerosil® 200 intensiv in einem Stephan® Mischer (Granulator) gemischt. Durch Befeuchten mit Wasser entstand eine leicht handfeuchte Mischung. Diese wird in einem Glatt® Wirbelschichtgranulator mit Wurster-Rohr unter 50°C Zuluft zunächst getrocknet und anschließend durch Bottom-Spraying mit einer Lösung von 110 g HPMC vom Typ Pharmacoat® 603, Firma Shinetsu, in 2 kg destilliertem Wasser bei einer Sprühmenge von 75 g Lösung pro Minute bei 50°C Zuluft besprüht. Nach Sprühende wurde bis zu einer Produkttemperatur von 38°C abgetrocknet und das Gut durch ein Sieb (Frewitt®) von 0,8 mm Maschenweite getrieben.

Zusammensetzung der Wirkstoffzubereitung 1.1.2, bezogen auf das Trockengewicht:

| | |
|---|---|
| Ibuprofen | 95,0 Gew.-% |
| Hydroxypropylmethylcellulose | 4,4 Gew.-% |
| Aerosil 200 | 0,6 Gew.-% |

Analog wurde eine Wirkstoffzubereitung 1.1.2.A hergestellt, bestehend aus 98,5 Gew.-% Ibuprofen und 1,5 Gew.-% HPMC ohne Aerosil 200.

Analog wurde eine Wirkstoffzubereitung 1.1.2.B hergestellt, bestehend aus 97,5 Gew.-% Ibuprofen und 2,5 Gew.-% HPMC ohne Aerosil 200.

### 1.2 Hydroxypropylcellulose (HPC) als Bindemittel

962 g Ibuprofen (feines Pulver) wurden mit 30 g Hydroxypropylcellulose vom Typ Klucel®, Firma Hercules Corp., Milldate, Conn., USA und 8 g Aerosil® 200 intensiv in einem Labor-Stephan® Mischer (Granulator) gemischt und unter leichtem Rühren der Rühreinrichtung 1 mit 130 g destillierten Wasser befeuchtet und weitere 5 Minuten intensiv gerührt. Danach wurde das feuchte Gut durch ein Sieb der Maschenweite 3 bis 4 mm getrieben und in einem Wirbelschichttrockner (Fa. Glatt) bei einer Zulufttemperatur von 50 bis 60°C getrocknet. Es erfolgte eine Siebung mit Sieben kleiner 800 µm. Nach einer Laserbeugungspartikelmesung waren Agglomerate unter 80 bis 90 µm nur in wenigen Prozentanteilen von 3 bis 6 % enthalten.

Zusammensetzung der Wirkstoffzubereitung 1.2, bezogen auf das Trockengewicht:

| | |
|---|---|
| Ibuprofen | 96,2 Gew.-% |
| Hydroxypropylcellulose | 3,0 Gew.-% |
| Aerosil 200 | 0,8 Gew.-% |

Analog wurde eine Wirkstoffzubereitung 1.2.A hergestellt, bestehend aus 98 Gew.-% Ibuprofen und 2 Gew.-% HPC ohne Aerosil 200.

### 1.3 Gelatine als Bindemittel

4,5 kg Ibuprofen (feines Pulver) wurden mit 25 g Aerosil® 200 intensiv in einem Diosna® Mischer (Granulator) gemischt. Unter leichtem Rühren des Bodenpropellers und des Zerhackers bei Stufe 1 wurde mit 500 g destilliertem Wasser befeuchtet und weitere 5 Minuten intensiv gerührt, bis eine nicht mehr trennbare Mischung von Ibuprofen und Aerosil entstand. Danach wurde das feuchte Gut in einen Wirbelschichtgranulator des Typ Fa. Glatt gegeben und mit einer 50°C heißen wäßrigen Lösung von 225 g Gelatine in 3,525 kg Wasser bei einer Zulufttemperatur von 60°C in Sprühmengen von 150 g/Minute aufgesprüht. Nach Sprühende wurde bis zu einer Produkttemperatur von 38°C abgetrocknet und das Gut durch ein Sieb (Frewitt®) von 0,8 mm Maschenweite getrieben.

Zusammensetzung der Wirkstoffzubereitung 1.3, bezogen auf das Trockengewicht:

| | |
|---|---|
| Ibuprofen | 94,74 Gew.-% |
| Gelatine | 4,74 Gew.-% |
| Aerosil 200 | 0,52 Gew.-% |

Analog wurde eine Wirkstoffzubereitung 1.3.A hergestellt bestehend aus 99 Gew.-% Ibuprofen und 1 Gew.-% Gelatine ohne Aerosil 200.

### 1.4 Natriumcarboxymethylcellulose (NaCMC) als Bindemittel

9,78 Kg Ibuprofen (feines Pulver, Sorte 25 der Firma Knoll AG, Ludwigshafen) wurden mit 20 g Aerosil 200 und 200 g Tylose® C (Na-Carboxymethylcellulose der Firma Hoechst, Frankfurt) in einem Stephan-Labormischer intensiv miteinander gemischt und solange in kleinen Mengen Wasser zugegeben, bis eine erdfeuchte Masse entstand (ca. 1,5 bis 2,0 1 Wasser). Über ein Sieb mit 3,15 mm Maschenweite (Frewitt®) wurde die Masse zerkleinert und in einem Glatt®-Wirbelschichttrockner bei 50°C Zuluft getrocknet. Anschließend wurde das trockene Produkt erneut durch ein Sieb mit 0,8 mm Maschenweite (Frewitt^{(R)}) getrieben.

Zusammensetzung der Wirkstoffzubereitung 1.4, bezogen auf das Trockengewicht:

| | |
|---|---|
| Ibuprofen | 97,8 Gew.-% |
| NaCMC | 2,0 Gew.-% |
| Aerosil 200 | 0,2 Gew.-% |

Analog wurde eine Wirkstoffzubereitung 1.4.A hergestellt bestehend aus 98,5 Gew.-% Ibuprofen und 1,5 Gew.-% NaCMC ohne Aerosil 200.

### 1.5 Methylcellulose als Bindemittel

4,275 kg Ibuprofen 38 (Knoll AG) wurden mit einer Lösung von 0,203 kg Methylcellulose in 5 kg dest. Wasser in der Wirbelschicht bei 50°C Zulufttemperatur bei einer Sprührate von ca. 90.g/Minute über etwa eine Stunde agglomeriert mit einer Abrüttelphase von ca. 30 Sekunden alle 3 Minuten. Nach Trocknung bei Erreichen einer Ablufttemperatur von ca. 40°C wird 22g Aerosil 200 zugemischt und das Produkt durch ein Sieb mit 0,8 mm Maschenweite gedrückt.

Zusammensetzung der Wirkstoffzubereitung 1.5, bezogen auf das Trockengewicht:

| | |
|---|---|
| Ibuprofen | 95 Gew.-%, |
| Methylcellulose | 4,51 Gew.-%, |
| Aerosil 200 | 0,49 Gew.-%, |

Analog wurde eine Wirkstoffzubereitung 1.5.A hergestellt bestehend aus 98 Gew.-% Ibuprofen und 2 Gew.-% Methylcellulose ohne Aerosil 200.

Analog wurde eine Wirkstoffzubereitung 1.5.B hergestellt bestehend aus 96 Gew.-% Ibuprofen und 4 Gew.-% Methylcellulose ohne Aerosil 200.

### Beispiel 2

Herstellung von Preßlingen aus der Wirkstoffzubereitung und Vergleich der Daten mit Preßlingen die aus physikalischen Mischungen der gleichen Zusammensetzung hergestellt wurden.

Verschiedene Zusammensetzungen der erfindungsgemäßen Wirkstoffzubereitung wurden mit einer Presse, Typ EKO, Firma Korsch, Reclin zu Preßlingen von 200 mg mit einem Durchmesser von 9 mm, bei unterschiedlichen Drücken gepreßt. Im Vergleich dazu wurden die physikalischen Mischungen (PM) mit gleicher Zusammensetzung unter analogen Bedingungen gepreßt und die Härten der hergestellten Preßlinge verglichen. Unter physikalischen Mischungen wird die trockene Mischung von Ibuprofen mit dem jeweiligen Bindemittel verstanden.

Die Ergebnisse sind in der nachstehenden Tabelle aufgelistet. Im Gegensatz zu den rein physikalischen Mischungen führen die erfindungsgemäßen ibuprofenhaltigen Wirkstoffzusammensetzungen auch schon bei geringen Preßkräften zu Preßlingen mit Härten, die deutlich über den für Tabletten ausreichenden Härten von 25 N liegen.

**Tabelle:**

| Abhängigkeit der Härte vom Ibuprofenpreßlingen vom jeweiligen Preßdruck und vom Vergleich erfindungsgemäße Wirkstoffzubereitung (WZ) und physikalische Mischung (PM) | | | | |
|---|---|---|---|---|
| Zusammensetzung | Präparation nach Beispiel | Härte in [N] bei einer Preßkraft von | | |
| Ibuprofen mit Menge Bindemittel in [Gew.-%] bezogen auf Zusammensetzung | | 4,7 bis 5,3 kN | 9,7 bis 10,9 kN | 20,1 bis 21,1 kN |
| 0% (rein) | - | 11,0 | 10,8 | 20,1 |
| 1,5 % HPMC | WZ nach 1.1.2.A | 63,0 | 120,0 | 165,0 |
| | PM | 13,0 | 18,0 | 30, 0 |
| 2,5 % HPMC | WZ nach 1.1.2.B | 70,0 | 131,0 | 160,0 |
| | PM | 15,0 | 19,0 | 29,0 |
| 2 % Methylcellulose | WZ nach 1.5.A | 85,0 | 145,0 | 153,0 |
| | PM | 9,0 | 16,0 | 38,0 |
| 4 % Methylcellulose | WZ nach 1.5.B | 98,0 | 148,0 | 175,0 |
| | PM | 14,0 | 20,0 | 29,0 |
| 2 % HPC | WZ nach 1.2.A | 65,0 | 98,0 | 156,0 |
| | PM | 18,0 | 21,0 | 35,0 |
| 1,5 % NaCMC | WZ nach 1.4.A | 98,0 | 130,0 | 180,0 |
| | PM | 8,0 | 15,0 | 28,0 |
| 1 % Gelatine | WZ nach 1.3.A | 53,0 | 98,0 | 121,0 |
| | PM | 7,0 | 12,0 | 30,0 |
| Abkürzungen: HPMC = Hydroxypropylmethylcellulose HPC = Hydroxypropylcellulose NaCMC = Natriumcarboxymethyl-cellulose | | | | |

### Beispiel 3

### Herstellung von Tabletten aus den Wirkstoffzubereitungen

Die unter Beispiel 1 hergestellten Wirkstoffzubereitungen wurden mit weiteren Hilfsstoffen und/oder weiteren wirkstoffen gemischt (Ansatzgröße ca. 1 kg) und auf einer instrumentierten Excenterpresse vom Typ EK 0 der Firma Korsch, Berlin direkt in Tabletten gepreßt. Die nachstehenden Tabellen in den Beispielen geben die Zusammensetzung der Arzneimittelzubereitung für eine Tablette und damit die Zusammensetzung der einzelnen Tablette an.

### 3.1 Ibuprofen-Tablette einer Dosis von 200 mg Wirkstoff aus der Wirkstoffzubereitung gemäß Beispiel 1.2

| Wirkstoffzubereitung gemäß Beispiel 1.2 | 208 mg (entspricht 200 mg Ibuprofen) |
|---|---|
| Lactose | 10,0 mg |
| prägelatinisierte Stärke | 15,0 mg |
| AcDiSol | 12,0 mg |
| Magnesiumstearat | 1,5 mg |
| Aerosil | 0,5 mg |

| | |
|---|---|
| Tablettengewicht | 247 mg |
| Stempelgröße | 9 mm rund |
| Wirkstoffanteil | 81 Gew.-% |
| Preßkraft | 6 kN |
| Härte | 89 N |
| Zerfall | 2 Minuten Becherglas mit Wasser bei Raumtemperatur |
| Wirkstoff-Freisetzung | 5 Minuten 78 % 10 Minuten 98 % (USP Methode) |

### 3.2 Ibuprofen-Tablette einer Dosis von 600 mg Wirkstoff aus der Wirkstoffzubereitung gemäß Beispiel 1.2

Werden Tabletten mit der gleichen Rezeptur wie in Beispiel 3.1 auf Oblong-Formaten von 6,5 x 18 mm und einem Gewicht von 741 mg gepreßt, erhält man bei einer Preßkraft von 7 kN Tabletten einer Härte von 158 N und einer Zerfallszeit von 2 Minuten in Wasser. Die Wirkstoff-Freisetzung nach USP XXII zeigt nach 10 Minuten 100 % gelöstes Ibuprofen.

### 3.3 Ibuprofen-Tablette einer Dosis von 600 mg Wirkstoff aus der Wirkstoffzubereitung gemäß Beispiel 1.1.1

| Wirkstoffzubereitung gemäß Beispiel 1.1.1 | 617 mg (entspricht 600 mg Ibuprofen) |
|---|---|
| Microkristalline Cellulose | 18,0 mg |
| Lactose | 6,0 mg |
| ÄcDiSol | 14,0 mg |
| Magnesiumstearat | 4,5 mg |
| Aerosil | 1,5 mg |

| | |
|---|---|
| Tablettengewicht | 661 mg |
| Stempelgröße | 6,5 x 18 mm oblong |
| Wirkstoffanteil | 91,1 Gew.-% |
| Preßkraft | 9 kN |
| Härte | 180 N |
| Zerfall | 4 Minuten Becherglas mit Wasser bei Raumtemperatur |
| Wirkstoff-Freisetzung | 5 Minuten 68 % 10 Minuten 89 % 15 Minuten 99 % (USP Methode) |

### 3.4 Ibuprofen-Tablette einer Dosis von 400 mg Wirkstoff aus der Wirkstoffzubereitung gemäß Beispiel 1.1.1

Werden Tabletten mit der gleichen Rezeptur wie in Beispiel 3.3 auf Stempel-Formaten von 11 mm, rund und einem Gewicht von 440,8 mg gepreßt, erhält man bei einer Preßkraft von 6 kN Tabletten einer Härte von 138 N und einer Zerfallszeit von 3 Minuten in Wasser. Die Wirkstoff-Freisetzung nach USP XXII zeigt nach 15 Minuten 100 % gelöstes Ibuprofen.

### 3.5 Kombinations-Tablette mit einer Dosis von 400 mg Ibuprofen und 10 mg Dihydrocodeinhydrogentatrat aus der ibuprofenhaltigen Wirkstoffzubereitung gemäß Beispiel 1.1.1

| Wirkstoffzubereitung gemäß Beispiel 1.1.1 | 411,5 mg (entspricht 400 mg Ibuprofen) |
|---|---|
| Dihydrococeinhydrogentartrat | 10 mg 10 mg |
| Microkristalline Cellulose | 25 mg |
| Lactose | 10 mg |
| Pharmacoat® 603 | 8 mg |
| AcDiSol | 12 mg |
| Magnesiumstearat | 3 mg |
| Aerosil | 1 mg |

| | |
|---|---|
| Tablettengewicht | 480,5 mg |
| Stempelgröße | 11 mm rund |
| Wirkstoffanteil | 86,3 Gew.-% |
| Preßkraft | 6 kN |
| Härte | 95N |
| Zerfall | 3 Minuten Becherglas mit Wasser bei Raumtemperatur |
| Wirkstoff-Freisetzung Ibuprofe | 5 Minuten 68 % 10 Minuten 89 % 15 Minuten 99 % (USP Methode) |

### 3.6 Ibuprofen-Tablette einer Dosis von 200, 400, 600 und 800 mg Wirkstoff aus der Wirkstoffzubereitung gemäß Beispiel 1.1.1 mit einem Wirkstoffanteil von 80 Gew.-% in der Tablette

| Wirkstoffzubereitung gemäß Beispiel 1.1.1 | 83,3 Gew.-% (entspricht 80 Gew.-% Ibuprofen) |
|---|---|
| mikrokristalline Cellulose | 4,3 Gew.-% |
| Lactose | 5,4 Gew.-% |
| AcDiSol | 6,0 Gew.-% |
| Aerosil 200 | 0,6 Gew.-% |
| Magensiumstearat | 0,4 Gew.-% |

Die aus diesen Bestandteilen gemischten Arzneimittelzubereitung wurden zu Tabletten gepreßt mit einem Gewicht von 250 mg (= 200 mg Ibuprofen), 500 mg (= 400 mg Ibuprofen), 750 mg (= 600 mg Ibuprofen) und 1000 mg (= 800 mg Ibuprofen) in Tablettengrößen von 9 mm rund, 11 mm rund, 6,5 x 18 mm und 7,5 x 21 mm.
Alle Tabletten setzten den Wirkstoff Ibuprofen in 10 Minuten quantitativ frei.

## Patentansprüche

1. Verfahren zur Herstellung einer Wirkstoffzubereitung, enthaltend Ibuprofen, **dadurch gekennzeichnet, daß** man Ibuprofen oder dessen Salze mit Hydroxymethylpropylcellulosen, Methylcellulose, Natriumcarboxymethylcellulose, Hydroxypropylcellulose oder Gelatine als Bindemittel überzieht, wobei bezogen auf das Trockengewicht der Wirkstoffzubereitung der Anteil des Bindemittels 0,1 bis 10 Gew.-% und der Anteil an Ibuprofen oder dessen Salze 90 bis 99,9 Gew.-% beträgt.

2. Verfahren nach Anspruch 1, **dadurch gekennzeichnet, daß** man dem zu überziehenden Ibuprofen oder dessen Salzen 0.1 bis 1,5 Gew.-% hochdisperses Siliciumdioxid zusetzt, bezogen auf das Trockengewicht der Wirkstoffzubereitung.

3. Wirkstoffzubereitung, erhältlich nach einem Verfahren gemäß einem der Ansprüche 1 oder 2.

4. Wirkstoffzuberereitung, bestehend aus
| | |
|---|---|
| 90,0 bis 99,9 Gew.-% | Ibuprofen oder dessen Salze, |
| 0,1 bis 10,0 Gew.-% | Hydroxymethylpropylcellulosen, Methylcellulose, Natriumcarboxymethylcellulose, Hydroxypropylcellulose oder Gelatine als Bindemittel und |
| 0,1 bis 1,5 Gew.-% | hochdispersem Siliciumdioxid, |
jeweils bezogen auf das Trockengewicht der Wirkstoffzubereitung, wobei
die aus dieser Wirkstoffzubereitung mit einer Preßkraft von 4,7 bis 5,3 kN hergestellten Preßlinge mit einem Ibuprofenwirkstoffgehalt von 200 mg und einem Durchmesser von 9 mm eine Härte von mindestens 25 N aufweisen.

5. Verwendung der Wirkstoffzubereitung gemäß Anspruch 3 oder 4 zur Herstellung von Arzneimittelzubereitungen oder festen Darreichungsformen.

6. Arzneimittelzubereitung, enthaltend eine Wirkstoffzubereitung gemäß Anspruch 3 oder 4.

7. Arzneimittelzubereitung nach Anspruch 6, **dadurch gekennzeichnet daß** die Arzneimittelzubereitung zusätzlich zur Wirkstoffzubereitung Tabletten-Hilfsstoffe enthält.

8. Arzneimittelzubereitung nach Anspruch 7, **dadurch gekennzeichnet daß** als Tabletten-Hilfsstoffe Stoffe enthalten sind, die eine verzögerte wirkstoffabgabe aus festen Darreichungsformen bewirken.

9. Arzneimittelzubereitung gemäß einem der Ansprüche 6 bis 8, die zusätzlich weitere Wirkstoffe enthält.

10. Verwendung der Arzneimittelzubereitungen gemäß einem der Ansprüche 6 bis 9 zur Herstellung von festen Darreichungsformen.

11. Verfahren zur Herstellung von festen Darreichungsformen, **dadurch gekennzeichnet, daß** man eine Wirkstoffzubereitung gemäß Anspruch 3 oder 4 oder eine Arzneimittelzubereitung gemäß einem der Ansprüche 6 bis 9 zu einer festen Form komprimiert und gegebenenfalls diese feste Darreichungsform mit Überzugsmitteln überzieht.

12. Feste Darreichungsformen, enthaltend eine Wirkstoffzubereitung gemäß Anspruch 3 oder 4.

13. Feste Darreichungsformen, enthaltend eine Arzneimittelzubereitung gemäß einem der Ansprüche 6 bis 9.

14. Feste Darreichungsformen nach Anspruch 12 oder 13 mit einem Ibuprofengehalt größer 75 Gew.-%.

## Claims

1. A process for producing an active ingredient preparation comprising ibuprofen, wherein ibuprofen or its salt is coated with hydroxypropylmethylcellulose, methylcellulose, sodium carboxymethylcellulose, hydroxypropylcellulose or gelatin as binder, where the proportion of the binder is from 0.1 to 10% by weight and the proportion of ibuprofen or its salts is from 90 to 99.9% by weight, based on the dry weight of the active ingredient preparation.

2. A process as claimed in claim 1, wherein from 0.1 to 1.5% by weight of highly disperse silica are added to the ibuprofen or its salts to be coated, based on the dry weight of the active ingredient preparation.

3. An active ingredient preparation obtainable by a process as claimed in either of claims 1 or 2.

4. An active ingredient preparation consisting of
| | |
|---|---|
| 90.0 to 99.9% | by weight of ibuprofen or its salts, |
| 0.1 to 10.0% | by weight of hydroxypropylmethylcellulose, methylcellulose, sodium carboxymethylcellulose, hydroxypropylcellulose or gelatin as binder and |
| 0.1 to 1.5% | by weight of highly disperse silica, |
in each case based on the dry weight of the active ingredient preparation, where
the compacts produced from this active ingredient preparation with a compressive force of from 4.7 to 5.3 kN and having an ibuprofen active ingredient content of 200 mg and a diameter of 9 mm have a hardness of at least 25 N.

5. The use of the active ingredient preparation as claimed in claim 3 or 4 for producing pharmaceutical preparations or solid dosage forms.

6. A pharmaceutical preparation comprising an active ingredient preparation as claimed in claim 3 or 4.

7. A pharmaceutical preparation as claimed in claim 6, wherein the pharmaceutical preparation comprises tablet excipients in addition to the active ingredient preparation.

8. A pharmaceutical preparation as claimed in claim 7, wherein the substances present as tablet excipients bring about delayed release of active ingredient from solid dosage forms.

9. A pharmaceutical preparation as claimed in any of claims 6 to 8, which additionally comprises further active ingredients.

10. The use of the pharmaceutical preparations as claimed in any of claims 6 to 9 for producing solid dosage forms.

11. A process for producing solid dosage forms, which comprises compressing an active ingredient preparation as claimed in claim 3 or 4 or a pharmaceutical preparation as claimed in any of claims 6 to 9 to a solid form and, where appropriate, coating this solid dosage form with coating agents.

12. A solid dosage form comprising an active ingredient preparation as claimed in claim 3 or 4.

13. A solid dosage form comprising a pharmaceutical preparation as claimed in any of claims 6 to 9.

14. A solid dosage form as claimed in claim 12 or 13 with an ibuprofen content of more than 75% by weight.

## Revendications

1. Procédé de préparation d'une composition de substance active, contenant de l'ibuprofène, **caractérisé en ce qu'**on revêt de l'ibuprofène ou ses sels par des hydroxyméthylpropylcelluloses, de la méthylcellulose, de la carboxyméthylcellulose sodique, de l'hydroxypropylcellulose ou de la gélatine, comme liant, la fraction du liant étant, par rapport au poids à l'état sec de la composition de substance active, de 0,1 à 10% en poids et la fraction d'ibuprofène ou de ses sels de 90 à 99,9% en poids.

2. Procédé suivant la revendication 1, **caractérisé en ce qu'**à l'ibuprofène ou ses sels à revêtir, on ajoute 0,1 à 1,5% en poids de dioxyde de silicium hautement dispersé, par rapport au poids à l'état sec de la composition de substance active.

3. Composition de substance active, que l'on peut obtenir suivant un procédé selon l'une des revendications 1 et 2.

4. Composition de substance active, constituée
de 90,0 à 99,9% en poids d'ibuprofène ou de ses sels,
de 0,1 à 10,0% en poids d'hydroxyméthylpropylcelluloses, de méthylcellulose, de carboxyméthylcellulose sodique, d'hydroxypropylcellulose ou de gélatine, comme liant, et
de 0,1 à 1,5% en poids de dioxyde de silicium hautement dispersé,
chaque fois par rapport au poids à l'état sec de la composition de substance active,
les comprimés préparés à partir de cette composition de substance active avec une force de pressage de 4,7 à 5,3 kN et avec une teneur en substance active d'ibuprofène de 200 mg et un diamètre de 9 mm présentant une dureté d'au moins 25 N.

5. Utilisation de la composition de substance active suivant l'une des revendications 3 et 4, pour la fabrication de compositions médicamenteuses ou de formes d'administration solides.

6. Composition médicamenteuse, contenant une composition de substance active suivant l'une des revendications 3 et 4.

7. Composition médicamenteuse suivant la revendication 6, **caractérisée en ce que** la composition médicamenteuse contient en supplément de la composition de substance active des adjuvants pour comprimés.

8. Composition médicamenteuse suivant la revendication 7, **caractérisée en ce que**, comme adjuvants pour comprimés, sont contenues des substances qui produisent une libération retardée de la substance active à partir de formes d'administration solides.

9. Composition médicamenteuse suivant l'une des revendications 6 à 8, qui contient en supplément d'autres substances actives.

10. Utilisation des compositions médicamenteuses suivant l'une des revendications 6 à 9, pour la préparation de formes d'administration solides.

11. Procédé de préparation de formes d'administration solides, **caractérisé en ce qu'**on comprime une composition de substance active suivant l'une des revendications 3 et 4 ou une composition médicamenteuse suivant l'une des revendications 6 à 9 en une forme solide et **en ce qu'**on revêt éventuellement cette forme d'administration solide de produits de revêtement.

12. Formes d'administration solides, contenant une composition de substance active suivant l'une des revendications 3 et 4.

13. Formes d'administration solides, contenant une composition médicamenteuse suivant l'une des revendications 6 à 9.

14. Formes d'administration solides suivant l'une des revendications 12 et 13, présentant une teneur en ibuprofène supérieure à 75% en poids.
